# EUROPEAN PATENT APPLICATION

(11) **EP 2 140 851 A1**
(43) Date of publication of application: **06.01.2010**
(21) Application number: 08751603.5
(22) Date of filing: 24.04.2008
(51) Int. Cl.: A61H 33/12, A45D 44/22, A61F 9/007, A61M 11/00, B05B 5/057

(54) **ELECTROSTATIC ATOMIZER**

(30) Priority: 24.04.2007 JP 2007114124
(71) Applicant: Daikin Industries, Ltd., Osaka-shi, Osaka 530-8323 (JP)
(72) Inventor: OBATA, Kouei, Sakai-shi Osaka 591-8511 (JP)
(74) Representative: HOFFMANN EITLE
(86) International application number: PCT/JP2008/001078
(87) International publication number: WO 2008/132848

(57) **Abstract**

An electrostatic spraying device (10) includes a container (15) for storing a liquid containing an ingredient valuable for a human body, a nozzle (20) communicating with the inside of the container (15), and an electric field generation section (31) to which a voltage is applied, thereby generating an electric field at a tip portion of the nozzle (20). The electrostatic spraying device (10) sprays the liquid from a tip of the nozzle (20) by generating the electric field at the tip portion of the nozzle (20) by using the electric field generation section (31). The electrostatic spraying device (10) is configured such that the direction of the nozzle (20) is changeable.

## Description

### TECHNICAL FIELD

The present invention relates to electrostatic spraying devices for spraying a liquid from a tip of a nozzle by generating an electric field at the tip portion of the nozzle.

### BACKGROUND ART

Electrostatic spraying devices for atomizing and spraying a liquid by Electro Hydrodynamics (EHD) have been known. Electrostatic spraying devices of this type are configured to generate an electric field at the tip portion of the nozzle, and atomize and spray the liquid at the tip of the nozzle by utilizing non-uniformity of the electric field. An electrostatic spraying device of this type is disclosed in Patent Document 1.

Specifically, the electrostatic spraying device of Patent Document 1 includes a solution tank, a spray nozzle, and a ring electrode. An aqueous solution of such as theanine, which is one of amino acids, is stored in the solution tank. The spray nozzle communicates with the interior space of the solution tank. The ring electrode is electrically insulated from the spray nozzle. In this electrostatic spraying device, when the power is turned on, a potential difference is applied between the ring electrode and the spray nozzle, thereby generating an electric field at the tip portion of the spray nozzle, and as a result, the liquid is sprayed from the tip of the spray nozzle.

### CITATION LIST

NON-PATENT DOCUMENT 1: Japanese Laid-Open Patent Application Publication No. 2006-231237

### SUMMARY OF THE INVENTION

### TECHNICAL PROBLEM

Incidentally, the direction of the nozzle is unchangeable in the conventional electrostatic spraying devices for providing droplets to persons. Thus, if the location of the user changes during the use of the electrostatic spraying device, the direction of the nozzle cannot be adjusted to the user unless the electrostatic spraying device itself is moved. For example, in the case where the electrostatic spraying device is fixed, it takes time and effort for the user to move the electrostatic spraying device. If the nozzle is not oriented toward the user, the sprayed droplets cannot efficiently reach the user.

The present invention was made in view of the above, and its object is to enable the direction of the nozzle to be easily adjusted to the user in an electrostatic spraying device for spraying a liquid from a tip of the nozzle by generating an electric field at the tip portion of the nozzle.

### SOLUTION TO THE PROBLEM

The first aspect of the present invention is an electrostatic spraying device including a container (15) for storing a liquid, a nozzle (20) communicating with an inside of the container (15), and an electric field generation section (31) to which a voltage is applied, thereby generating an electric field at a tip portion of the nozzle (20), wherein the electrostatic spraying device sprays the liquid from a tip of the nozzle (20) by generating the electric field at the tip portion of the nozzle (20) by using the electric field generation section (31). In this electrostatic spraying device, a liquid containing an ingredient valuable for a human body is stored in the container (15), and a direction of the tip of the nozzle (20) is changeable.

The second aspect of the present invention is that the first aspect of the present invention includes a location detection means (42, 43) configured to detect a location of a user, and a direction adjusting means (41, 44) configured to move the nozzle (20) such that the tip of the nozzle (20) is oriented toward the location of the user which the location detection means (42, 43) has detected.

The third aspect of the present invention is that in the second aspect of the present invention, the location detection means (42, 43) detects a location of the user's face, and the direction adjusting means (41, 44) moves the nozzle (20) such that the tip of the nozzle (20) is oriented toward the location of the user's face which the location detection means (42, 43) has detected.

The fourth aspect of the present invention is that the first aspect of the present invention includes a processing means (33) configured to have a personal computer (16) carry out processing for detecting a location of a user, and a direction adjusting means (26, 41) configured to move the nozzle (20) such that the tip of the nozzle (20) is oriented toward the location of the user which is obtained from the personal computer (16).

The fifth aspect of the present invention is that the first aspect of the present invention further includes a main body (11) in which the container (15) is provided, wherein the nozzle (20) is movably attached to the main body (11) so that the direction of the tip of the nozzle (20) is changeable.

The sixth aspect of the present invention is that the first aspect of the present invention includes a nozzle attachment section (11) to which the nozzle (20) is attached, and a movable support section (32) which movably supports the nozzle attachment section (11) so that the direction of the tip of the nozzle (20) is changeable.

-Operation-According to the first aspect of the present invention, an electrostatic spraying device (10) for spraying a liquid which contains an ingredient valuable for persons is configured such that the direction of a nozzle (20) is changeable. The direction of the nozzle (20) is adjusted manually or automatically. Thus, even if the location of the user changes during the use of the electrostatic spraying device (10), the direction of the nozzle (20) can be adjusted according to the location of the user.

According to the second aspect of the present invention, a location detection means (42, 43) detects the location of the user. Then, a direction adjusting means (41, 44) moves the nozzle (20) such that the tip of the nozzle (20) is oriented toward the location of the user which the location detection means (42, 43) has detected. According to the second aspect of the present invention, the direction of the nozzle (20) is automatically adjusted such that the tip of the nozzle (20) is oriented toward the user.

According to the third aspect of the present invention, the location detection means (42, 43) detects the location of the user's face. Then, the nozzle (20) is moved by the direction adjusting means (41, 44) such that the tip of the nozzle (20) is oriented toward the location of the user's face which the location detection means (42, 43) has detected.

According to the fourth aspect of the present invention, processing for detecting the location of the user is carried out by a personal computer (16). The electrostatic spraying device (10) is capable of communication, and is used, with a connection cable connecting between the electrostatic spraying device (10) and the personal computer (16). The direction adjusting means (26, 41) moves the nozzle (20) such that the tip of the nozzle (20) is oriented toward the location of the user which is obtained from the personal computer (16). According to the fourth aspect of the present invention, the direction of the nozzle (20) is automatically adjusted such that the tip of the nozzle (20) is oriented toward the user.

According to the fifth aspect of the present invention, the nozzle (20) is movably attached to the main body (11) in which a container (15) is provided. The nozzle (20) itself is moved to adjust the direction of the nozzle (20).

According to the sixth aspect of the present invention, the nozzle attachment section (11) to which the nozzle (20) is attached is movably supported by the movable support section (32). The direction of the nozzle (20) is adjusted by moving the nozzle attachment section (11).

### ADVANTAGES OF THE INVENTION

According to the present invention, the electrostatic spraying device (10) is configured such that the direction of the nozzle (20) is changeable, and therefore, even if the location of the user changes during the use of the electrostatic spraying device (10), the direction of the nozzle (20) can be adjusted according to the location of the user. Thus, it is not necessary to move the electrostatic spraying device (10) itself in order to adjust the direction of the nozzle (20). To move the electrostatic spraying device (10), attachment and detachment of the electrostatic spraying device (10) may be necessary. Thus, adjustment of the direction of the nozzle (20) enables the nozzle (20) to be oriented toward the user relatively easily.

According to the second and fourth aspects of the present invention, the direction of the nozzle (20) is automatically adjusted such that the tip of the nozzle (20) is oriented toward the user. The user therefore does not need to adjust the direction of the nozzle (20). Thus, the sprayed droplets can efficiently reach the user without the user's adjustment of the direction of the nozzle (20).

According to the third aspect of the present invention, the direction of the nozzle (20) is automatically adjusted such that the tip of the nozzle (20) is oriented toward the user's face. Thus, in the case where an ingredient valuable for faces, such as a cosmetic liquid, is used as a liquid to be sprayed, the sprayed droplets can efficiently reach the user's face without the user's adjustment of the direction of the nozzle (20).

According to the fourth aspect of the present invention, processing for detecting the location of the user is carried out by a personal computer (16). The electrostatic spraying device (10) therefore does not need to include a structure for detecting the location of the user, and thus, the structure of the electrostatic spraying device (10) can be simplified.

### BRIEF DESCRIPTION OF THE DRAWINGS

[FIG. 1] FIG. 1 is a schematic view of an electrostatic spraying device according to Embodiment 1 of the present invention.
[FIG. 2] FIG. 2 is a front view of the electrostatic spraying device according to Embodiment 1 of the present invention.
[FIG. 3] FIG. 3 is a diagrammatic view illustrating the situation where the electrostatic spraying device according to Embodiment 1 of the present invention is used.
[FIG. 4] FIG. 4 is a schematic view of a modification of the electrostatic spraying device according to Embodiment 1 of the present invention.
[FIG. 5] FIG. 5 is a schematic view of an electrostatic spraying device according to Embodiment 2 of the present invention.
[FIG. 6] FIG. 6 is a schematic view of a modification of the electrostatic spraying device according to Embodiment 2 of the present invention.

### DESCRIPTION OF REFERENCE CHARACTERS

- 10: electrostatic spraying device
- 11: casing (main body, nozzle attachment section)
- 15: container
- 16: personal computer
- 20: nozzle (spray nozzle)
- 31: electric field generation section
- 32: movable support section
- 33: program (processing means)
- 41: drive member (direction adjusting means)
- 42: imaging section (location detection means)
- 43: location calculation section (location detection means)
- 44: movement control section (direction adjusting means)

### DESCRIPTION OF EMBODIMENTS

Embodiments of the present invention will be described below based on the drawings.

«Embodiment 1 of the invention» Embodiment 1 of the present invention is described. In Embodiment 1, an electrostatic spraying device (10) according to the present invention is described. The electrostatic spraying device (10) is for supplying a cosmetic liquid to the user's face. To use the electrostatic spraying device (10), the electrostatic spraying device (10) is placed at a location from which droplets can reach the user by utilizing only an attractive force caused by an electric field.

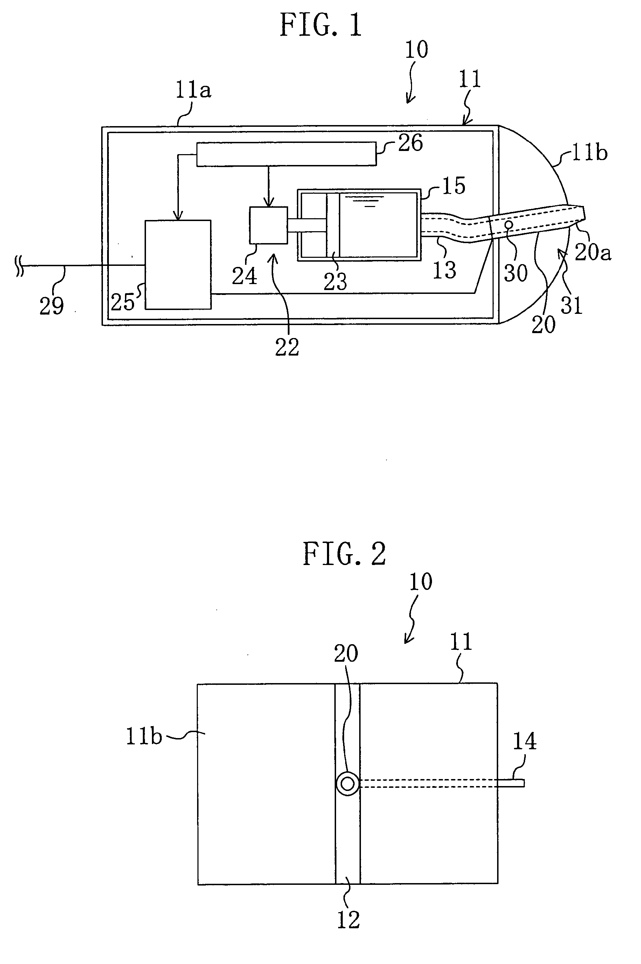

The electrostatic spraying device (10) includes a box-like casing (11) having a round front surface, as shown in FIG. 1. The casing (11) constitutes a main body and includes a rectangular parallelepiped shaped main section (11a) and a front surface section (11b) at the front of the main section (11a). The side surface of the main section (11a) and the side surface of the front surface section (11b) are flush with each other. The front of the front surface section (11b) forms an arc which extends from the upper front edge of the main section (11a) to the lower front edge of the main section (11a). As shown in FIG. 2, the front surface section (11b) has a groove (12) at the transverse center. The groove (12) extends from the upper edge to the lower edge of the front surface section (11b).

A container (15), a spray nozzle (20), a liquid transfer section (22), a power source section (25), and an operation control section (26) are provided in the casing (11). The container (15), the liquid transfer section (22), the power source section (25), and the operation control section (26) are accommodated within the main section (11a). The spray nozzle (20) is provided in the front surface section (11b).

The container (15) is for storing a liquid to be sprayed. The container (15) is cylinder-shaped. One end of the container (15) has a through hole for connecting a connection tube (13) with the container (15), and the other end of the container (15) has a through hole for inserting a rod of the liquid transfer section (22) described later. As a liquid valuable for the human body, a cosmetic liquid which contains a moisturizing ingredient and an antioxidant ingredient (for example, a liquid containing hyaluronic acid) is stored in the container (15). The concentration of the cosmetic liquid is controlled such that the electrical resistance rate is in a range of 1.0×10⁴ Ω cm to 1.0×10⁷ Ω cm, both inclusive.

The spray nozzle (20) is a nozzle (20) for spraying a liquid, and is made of a stainless circular pipe. A stainless circular pipe whose inner diameter is 0.2 mm is used for the spray nozzle (20). The tip portion (20a) of the spray nozzle (20) is formed such that its outer diameter decreases as it gets closer to the tip end. The outer peripheral surface of the tip portion (20a) is a conical surface whose apex angle is twenty degrees. The spray nozzle (20) communicates with the inside of the container (15) via the connection tube (13).

The spray nozzle (20) is provided in the groove (12) of the front surface section (11b) of the casing (11). The spray nozzle (20) is rotationally movably supported at the front surface section (11b) by a pin member (30) which is fit into the base end of the spray nozzle (20). The spray nozzle (20) is movably attached to the casing (11), i.e., the main body, so that the direction of the tip of the spray nozzle (20) can be changed.

Further, an adjustment lever (14) for adjusting the direction of the spray nozzle (20) is connected to the spray nozzle (20). The adjustment lever (14) is connected to a portion of the spray nozzle (20) that is located closer to the tip than the pin member (30). The adjustment lever (14) extends laterally and protrudes from the side surface of the front surface section (11b). The adjustment lever (14) is made of an insulating material. The user (P) of the electrostatic spraying device (10) can adjust the direction of the spray nozzle (20) in a vertical direction, i.e., the angle of the spray nozzle (20), by vertically moving the adjustment lever (14).

The liquid transfer section (22) is a so-called syringe pump. The liquid transfer section (22) includes a piston (23) for increasing the pressure inside the container (15), and a motor (24) for moving the piston. The piston (23) has a discoidal shape and is provided in the container (15) such that the periphery of the piston (23) is in close contact with the inner surface of the container (15). A space in the container (15) that is closer to the front than the piston (23) is filled with a liquid. The rear surface of the piston (23) is connected to the motor (24) via a rod.

In the liquid transfer section (22), the piston (23) gradually moves forward when the motor (24) is energized. When the piston (23) moves forward, the liquid in the container (15) is pushed out and supplied to the spray nozzle (20). The liquid transfer section (22) is configured to supply, for example, 1.5 µL of liquid per second to the spray nozzle (20). The amount of liquid which is supplied from the liquid transfer section (22) to the spray nozzle (20) is equal to the amount of liquid to be sprayed by the spray nozzle (20). The liquid transfer section (22) only needs to be configured such that the amount of liquid supplied to the spray nozzle (20) is in a range of 0.1 µL per second to 1.5 µL per second, both inclusive.

The power source section (25) is connected to a power cord (29) having a power plug. The power source section (25) is configured to supply power that is supplied through the power cord (29) when the power plug is inserted in a receptacle, to the motor (24) and the operation control section (26). Further, the power source section (25) is configured to convert the voltage (100 V) of a current supplied from the power cord (29) to a high voltage, such as 6 kV and apply the high voltage to the spray nozzle (20). The power source section (25) only needs to be configured to convert the voltage of the current supplied from the power cord (29) to a voltage of from 5 kV to 11 kV, both inclusive.

The operation control section (26) is a section for controlling the operation of the electrostatic spraying device (10). The operation control section (26) is configured to energize the motor (24) of the liquid transfer section (22) and at the same time to provide commands to the power source section (25) to make the power source section (25) apply a voltage to the spray nozzle (20), when the power of the electrostatic spraying device (10) is turned on.

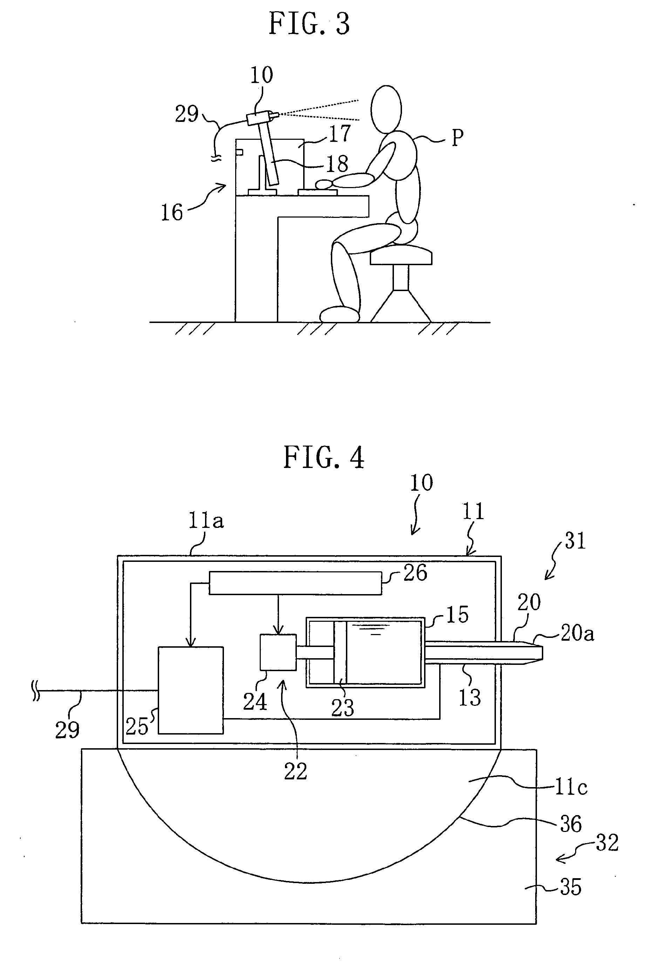

- Operation - The operation of the electrostatic spraying device (10) according to Embodiment 1 is described. To use the electrostatic spraying device (10), the electrostatic spraying device (10) is placed on the display (18) of the personal computer (16) as shown in FIG. 3. The casing (11) may be provided with a fixing member for fixing the electrostatic spraying device (10) to the display (18). To use the electrostatic spraying device (10), the electrostatic spraying device (10) may be mounted on the body (17) of the personal computer (16).

When the power of the electrostatic spraying device (10) is turned on, a voltage is applied to the spray nozzle (20) by the power source section (25), thereby generating an electric field at the tip portion of the spray nozzle (20), and at the same time, the motor (24) of the liquid transfer section (22) is energized. In Embodiment 1, the spray nozzle (20) itself constitutes an electric field generation section (31).

When the motor (24) is energized, the piston (23) gradually moves forward within the container (15), and thereby, the liquid in the container (15) is pushed out to be supplied to the spray nozzle (20). 1.5 µL of liquid per second is supplied to the spray nozzle (20). An electric filed is generated at the tip of the spray nozzle (20), and thus, the liquid in the spray nozzle (20) is polarized and positive electric charges gather at a location close to the air-liquid interface at the tip of the spray nozzle (20). At the tip of the spray nozzle (20), the air-liquid interface is drawn up to have a conical shape, and part of the aqueous solution is pulled apart from the top of the conical air-liquid interface as droplets. The amount of liquid that is equal to the amount of liquid supplied from the container (15) (1.5 µL per second) is sprayed from the spray nozzle (20).

If the magnitude of the applied voltage and the electrical resistance rate of the liquid are those in this Embodiment 1, the size of a droplet dispersed from the spray nozzle (20) is in a range of about 50 µm to 200 µm. The liquid dispersed from the spray nozzle (20) spreads toward the user (P) who is apart from the tip of the spray nozzle (20) by about forty to sixty centimeters, and adheres to the face of the user (P).

In Embodiment 1, the angle of the spray nozzle (20) can be manually adjusted by the adjustment lever (14). The user (P) can adjust the angle of the spray nozzle (20) in accordance with the height of the user's own face by operating the adjustment lever (14). The user (P) can adjust the angle of the spray nozzle (20) without touching the spray nozzle (20) to which a high voltage is applied.

- Effect of Embodiment 1-According to Embodiment 1, the electrostatic spraying device (10) is configured such that the direction of the spray nozzle (20) is changeable, and therefore, even if the location of the user changes during the use of the electrostatic spraying device (10), the direction of the spray nozzle (20) can be adjusted according to the location of the user. Thus, it is not necessary to move the electrostatic spraying device (10) itself in order to adjust the direction of the spray nozzle (20). To move the electrostatic spraying device (10), attachment and detachment of the electrostatic spraying device (10) may be necessary. Thus, adjustment of the direction of the nozzle (20) enables the nozzle (20) to be oriented toward the user relatively easily.

- Modification of Embodiment 1 - A modification of Embodiment 1 is described. An electrostatic spraying device (10) of this modification includes a casing (11) and a support member (35) which movably supports the casing (11), as shown in FIG. 4. The casing (11) constitutes a nozzle attachment section, and the support member (35) constitutes a movable support section (32).

Specifically, the casing (11) includes a rectangular parallelepiped shaped main section (11a) and a lower surface section (11c) provided on the lower surface of the main section (11a). The side surface of the main section (11a) and the side surface of the lower surface section (11c) are flush with each other. The lower surface of the lower surface section (11c) forms an arc which extends from the lower edge on the front side of the main section (11a) to the lower edge on the rear side of the main section (11a). In this modification, the spray nozzle (20) is fixed to the main section (11a).

The support member (35) is located under the casing (11). A hollow part (36) into which the lower surface section (11c) of the casing (11) is fit is formed in the upper surface of the support member (35). The hollow part (36) is arc-shaped. The diameter of the hollow part (36) is approximately equal to the diameter of the lower surface of the lower surface section (11c) of the casing (11).

The casing (11) is put on the support member (35), with the lower surface section (11c) fit in the hollow part (36). The casing (11) is rotationally movable along the circumference of the hollow part (36). When the casing (11) is rotationally moved, the direction of the spray nozzle (20) in a vertical direction, i.e., the angle of the spray nozzle (20), is changed. In this modification, the user (P) rotationally moves the casing (11) with the hands, and thereby, the angle of the spray nozzle (20) can be adjusted in accordance with the height of the face of the user (P).

«Embodiment 2 of the invention» Embodiment 2 of the present invention is described. In Embodiment 2, an electrostatic spraying device (10) according to the present invention is described. As in Embodiment 1, the electrostatic spraying device (10) is for supplying a cosmetic liquid to the user's face, and to use this electrostatic spraying device (10), the electrostatic spraying device (10) is placed at a location from which droplets can reach the user by utilizing only an attractive force caused by an electric field.

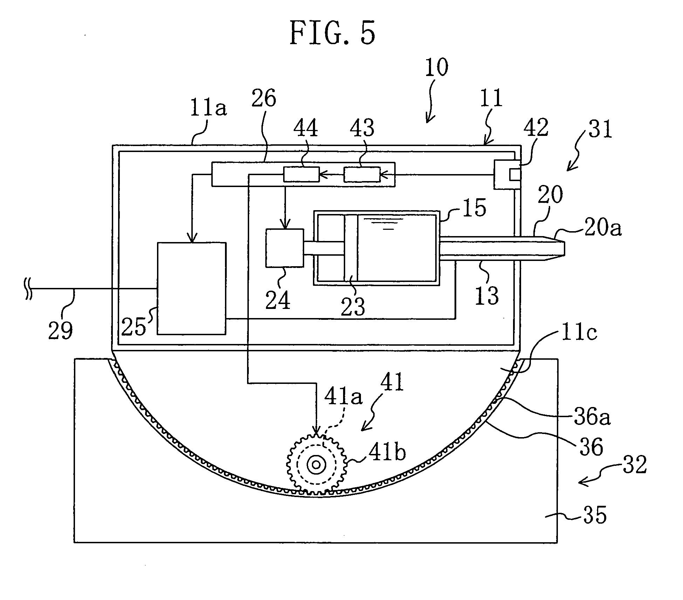

The electrostatic spraying device (10) includes a function for automatically adjusting the direction of the spray nozzle (20). As shown in FIG. 5, the electrostatic spraying device (10) includes a drive member (41), an imaging section (42), a location calculation section (43), and a movement control section (44), in addition to the structure of the electrostatic spraying device described in the above modification of Embodiment 1. The location calculation section (43) and the movement control section (44) are provided in the operation control section (26). The imaging section (42) and the location calculation section (43) constitute a location detection means. The drive member (41) and the movement control section (44) constitute a direction adjusting means.

The drive member (41) includes a drive motor (41a) and a gear wheel (41b) connected to the shaft of the drive motor (41a). The drive member (41) is provided at a lower portion of the lower surface section (11c) of the casing (11). The drive motor (41a) is fixed to the lower surface section (11c). The drive member (41) is provided such that only a tooth portion of the gear wheel (41b) protrudes from the lower surface of the lower surface section (11c).

An engagement portion (36a) which has projections and depressions and which engages with the gear wheel (41b) of the drive member (41) is formed in the hollow part (36) of the support member (35). The casing (11) is put on the support member (35) such that the gear wheel (41b) of the drive member (41) engages with the engagement portion (36a). When the drive motor (41a) is energized, the gear wheel (41b) rotates as it engages with the engagement portion (36a), and the casing (11) rotationally moves along the circumference of the hollow part (36).

The imaging section (42) is constituted by a CCD camera. The imaging section (42) is provided at the front of the main section (11a) of the casing (11). The imaging section (42) is provided such that the optical axis of the imaging section (42) aligns with the direction of the spray nozzle (20).

The location calculation section (43) is connected to the imaging section (42). Data of an image captured by the imaging section (42) is input to the location calculation section (43). The location calculation section (43) is configured to be capable of extracting the face of a person if the data of the image includes the face of the person, by utilizing an algorithm for extracting face images. The algorithm for extracting face images is created such that it extracts feature points from the data of the image, performs pattern matching based on the feature points, and thereby recognizes the face of the person.

The location calculation section (43) is configured to detect the location of the face in the image data if the image data includes the face of a person.

The movement control section (44) is configured to adjust the direction of the spray nozzle (20) such that the tip of the spray nozzle (20) is oriented toward the center of the user's face, by controlling the drive motor (41a) based on the location of the face in the image data that has been detected by the location calculation section (43).

Specifically, the amount of adjustment of the direction of the spray nozzle (20), the amount of adjustment corresponding to the location of the face in the image data, is stored in the movement control section (44) beforehand as a database. When the location calculation section (43) detects the location of the face in the image data, the movement control section (44) reads the amount of adjustment of the direction of the spray nozzle (20), the amount of adjustment corresponding to the location of the face in the image data, from the database. The movement control section (44) is configured to then output, to the drive motor (41a), the amount of operation of the drive motor (41a) based on the amount of adjustment of the direction of the spray nozzle (20). The rotational direction and the angle of rotation of the drive motor (41a) are output as the amount of operation of the drive motor (41a). When the drive motor (41a) is energized according to the amount of operation, the direction of the spray nozzle (20) is adjusted such that the tip of the spray nozzle (20) is oriented toward the center of the user's face.

According to the electrostatic spraying device (10) of Embodiment 2, the direction adjustment movement for adjusting the direction of the spray nozzle (20) is carried out at predetermined intervals (e.g., at intervals of ten seconds).

In the direction adjustment movement, first of all, the imaging section (42) captures an image in front of the electrostatic spraying device (10). Next, the location calculation section (43) analyzes the data of the image captured by the imaging section (42) and if the image data includes the face of a person, detects the location of the face in the image data. Next, the location calculation section (43) reads the amount of adjustment of the direction of the spray nozzle (20), the amount of adjustment corresponding to the location of the face in the image data, and outputs, to the drive motor (41a), the amount of operation of the drive motor (41a) based on the amount of adjustment of the direction of the spray nozzle (20). As a result, the drive motor (41a) is energized in accordance with the amount of operation, and the direction of the spray nozzle (20) is adjusted to be oriented toward the face of the user.

- Effect of Embodiment 2 - According to Embodiment 2, the direction of the spray nozzle (20) is automatically adjusted so that the tip of the spray nozzle (20) is oriented toward the user's face. The user therefore does not need to adjust the direction of the spray nozzle (20). Thus, the sprayed droplets can efficiently reach the user's face without the user's adjustment of the direction of the spray nozzle (20).

- Modification of Embodiment 2 - A modification of Embodiment 2 is described. In this modification, the processing until decision of the amount of adjustment of the direction of the spray nozzle (20) described in Embodiment 2, is carried out by a personal computer (16) to which the electrostatic spraying device (10) is connected. To use the electrostatic spraying device (10) of this modification, the electrostatic spraying device (10) of this modification is placed at a predetermined location with respect to the personal computer (16), for example, at the center of the upper surface of the display (18). Unlike Embodiment 2, the imaging section (42) and the location calculation section (43) are not provided in this electrostatic spraying device (10).

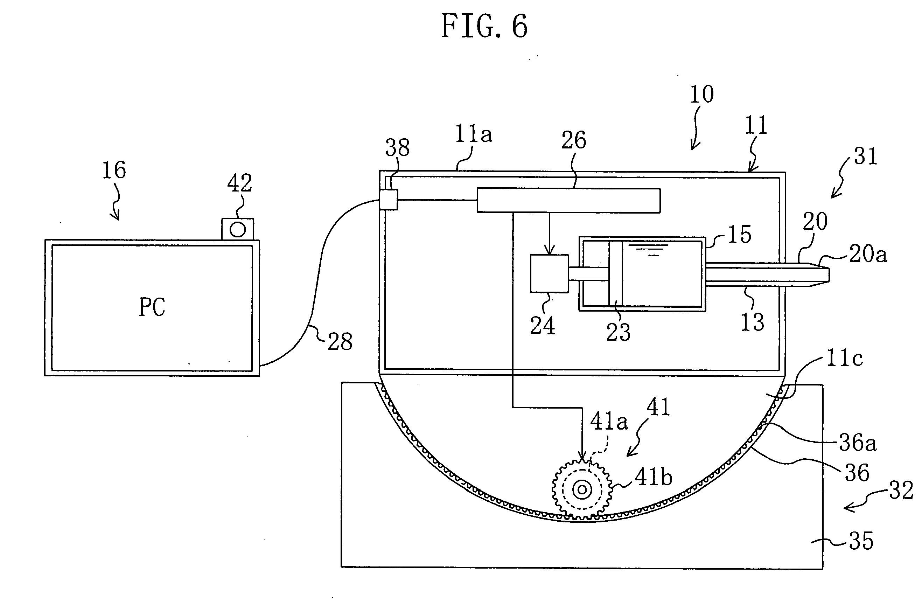

The personal computer (16) to which this electrostatic spraying device (10) is connected includes a CCD camera (42) as shown in FIG. 6. In addition, a program (33) for having the personal computer (16) carry out processing in which a location of the user's face is detected based on data of the image captured by the CCD camera (42) and in which the amount of adjustment of the direction of the spray nozzle (20) is decided based on the detected location of the face, is installed in the personal computer (16) as a processing means. The program (33) is configured to have the personal computer (16) carry out face extraction processing, location calculation processing, processing for deciding the amount of adjustment, and data output processing. The positional relationship between the CCD camera (42) and the spray nozzle (20) is input in the personal computer (16).

The face extraction processing is processing in which data of an image captured by the CCD camera (42) is obtained and in which whether or not the face of a person is included in the data of the image is determined. The earlier described algorithm for extracting face images is used in this face extraction processing.

The location calculation processing is processing in which the location of the face in the image data is detected if the face of a person is included in the image data in the face extraction processing.

The processing for deciding the amount of adjustment is processing in which the amount of adjustment of the direction of the spray nozzle (20) is decided based on the location of the face in the image data that has been detected in the location calculation processing. The program (33) includes the database described in Embodiment 2, and the database is used in the processing for deciding the amount of adjustment.

The data output processing is processing in which the amount of operation of the drive motor (41a) based on the amount of adjustment of the direction of the spray nozzle (20), the amount of adjustment having been decided in the processing for deciding the amount of adjustment, is output to the electrostatic spraying device (10). The amount of operation of the drive motor (41a) is the rotational direction and the angle of rotation of the drive motor (41a) at the time when the direction of the spray nozzle (20) is changed by the amount of adjustment.

The operation control section (26) of the electrostatic spraying device (10) is configured to be capable of communicating with the personal computer (16) via a connecting cord (28). The electrostatic spraying device (10) is provided with a connection terminal (38) to which the connecting cord (28), e.g., USB cord, connected to a connection terminal of the personal computer (16), e.g., USB port, is connected. The connection terminal (38) is connected to the operation control section (26). The operation control section (26) may be configured to be capable of communicating with the personal computer (16) by radio waves.

The operation control section (26) is configured to receive the amount of operation of the drive motor (41a) from the personal computer (16) and output the received amount of operation to the drive motor (41a). In this modification, the drive motor (41a) is energized according to the amount of operation which has been input to the drive motor (41a), and the direction of the spray nozzle (20) is adjusted to the user's face. The operation control section (26) and the drive member (41) constitute a direction adjusting means for moving the spray nozzle (20) such that the tip of the spray nozzle (20) is oriented toward the location of the user's face that is obtained from the personal computer (16).

In this modification, the detection of the location of the user's face and the decision of the amount of operation of the drive motor (41a) based on the location of the face are carried out by the personal computer (16). Thus, the structure of the electrostatic spraying device (10) can be simplified because the electrostatic spraying device (10) does not need to include the imaging section (42), the location calculation section (43), and the movement control section (44) described in Embodiment 2.

«Other Embodiments» The following structures may be applied in the above embodiments.

A liquid for eye drops may be used as the liquid to be sprayed in the above embodiments. In this case, the electrostatic spraying device (10) is used to relieve dry eyes. The electrostatic spraying device (10) is configured such that the amount of liquid to be sprayed from the spray nozzle (20) is in a range of 0.1 µL per second to 1.0 µL per second, both inclusive, to prevent valuable substances contained in tears from flowing with the liquid which adheres to the eyes.

In the above embodiments, the liquid to be sprayed may be a liquid containing an ingredient for protecting the mucosa of the throat.

In the above embodiments, the electrostatic spraying device (10) may be configured such that the direction of the spray nozzle (20) is adjustable not only in a vertical direction, but also in a horizontal direction. In the case of the electrostatic spraying device (10) described in the modification of Embodiment 1, the lower surface section (11c) of the drive motor (41a) and the hollow part (36) of the support member (35) are made to have a spherical surface. In the case of the electrostatic spraying device (10) described in Embodiment 2, a drive member for rotating the casing (11) in a horizontal direction is provided.

In the above embodiments, the electrostatic spraying device (10) may be configured such that the direction of the spray nozzle (20) is adjustable only in a horizontal direction.

In the above embodiments, to obtain electric power from the personal computer (16), the electrostatic spraying device (10) may be configured to be connectable to a connection terminal of the body (17) of the personal computer (16), e.g., a USB port and IEEE 1394, which are capable of supplying electric power. In this case, the user does not have to use a receptacle, and therefore, usability of the electrostatic spraying device (10) can be enhanced.

In the above embodiments, the electric field generation section (31) may be composed of two electrodes. In this case, one of the two electrodes is in contact with liquid in the spray nozzle (20), and the other electrode is insulated from the liquid in the spray nozzle (20). When a potential difference is applied between the two electrodes by the power source section (25), an electric field is generated at the tip portion of the spray nozzle (20).

In Embodiment 2, the electrostatic spraying device (10) may be configured such that the user of the personal computer (16) can adjust the direction of the spray nozzle (20) via the personal computer (16). Specifically, the electrostatic spraying device (10) is configured to be capable of communicating with the personal computer (16) as in the above modification of Embodiment 2. In addition, a program (33) for outputting to the electrostatic spraying device (10) a signal for adjusting the direction of the spray nozzle (20) according to keyboard operation is installed in the personal computer (16). When the user of the personal computer (16) presses, for example, a cursor key of the keyboard, the direction of the spray nozzle (20) is adjusted in the direction of the cursor key.

In Embodiment 2, the electrostatic spraying device (10) may have a remote controller for adjusting the direction of the spray nozzle (20).

The embodiments described in the above are essentially preferable examples which are not intended to limit the present invention, its application, or its range of use.

### Industrial Applicability

As described in the above, the present invention is useful as an electrostatic spraying device for spraying a liquid from a tip of a nozzle by generating an electric field at the tip portion of the nozzle.

## Claims

1. An electrostatic spraying device comprising:
a container (15) for storing a liquid;
a nozzle (20) communicating with an inside of the container (15); and
an electric field generation section (31) to which a voltage is applied, thereby generating an electric field at a tip portion of the nozzle (20),
wherein the electrostatic spraying device sprays the liquid from a tip of the nozzle (20) by generating the electric field at the tip portion of the nozzle (20) by using the electric field generation section (31),
a liquid containing an ingredient valuable for a human body is stored in the container (15), and
a direction of the tip of the nozzle (20) is changeable.

2. The electrostatic spraying device of claim 1, comprising:
a location detection means (42, 43) configured to detect a location of a user; and
a direction adjusting means (41, 44) configured to move the nozzle (20) such that the tip of the nozzle (20) is oriented toward the location of the user which the location detection means (42, 43) has detected.

3. The electrostatic spraying device of claim 2, wherein
the location detection means (42, 43) detects a location of the user's face, and
the direction adjusting means (41, 44) moves the nozzle (20) such that the tip of the nozzle (20) is oriented toward the location of the user's face which the location detection means (42, 43) has detected.

4. The electrostatic spraying device of claim 1, comprising:
a processing means (33) configured to have a personal computer (16) carry out processing for detecting a location of a user; and
a direction adjusting means (26, 41) configured to move the nozzle (20) such that the tip of the nozzle (20) is oriented toward the location of the user which is obtained from the personal computer (16).

5. The electrostatic spraying device of claim 1, further comprising:
a main body (11) in which the container (15) is provided, wherein
the nozzle (20) is movably attached to the main body (11) so that the direction of the tip of the nozzle (20) is changeable.

6. The electrostatic spraying device of claim 1, comprising:
a nozzle attachment section (11) to which the nozzle (20) is attached, and
a movable support section (32) which movably supports the nozzle attachment section (11) so that the direction of the tip of the nozzle (20) is changeable.
